# EUROPÄISCHE PATENTSCHRIFT

(11) **EP 1 525 175 B1**
(45) Veröffentlichungstag und Bekanntmachung des Hinweises auf die Patenterteilung: **09.07.2008**
(21) Anmeldenummer: 03766229.3
(22) Anmeldetag: 18.07.2003
(51) Int. Cl.: C07C 29/10

(54) **VERFAHREN ZUR AUSBEUTEERHÖHUNG BEI DER HERSTELLUNG VON MEHRWERTIGEN ALKOHOLEN DURCH SPALTUNG ACETALHALTIGER NEBENPRODUKTE**
METHOD FOR INCREASING YIELD IN THE PRODUCTION OF POLYVALENT ALCOHOLS BY SPLITTING BY-PRODUCTS CONTAINING ACETAL
PROCEDE POUR AUGMENTER LE RENDEMENT DANS LE CADRE DE LA PREPARATION D'ALCOOLS PLURIVALENTS PAR SEPARATION DE PRODUITS SECONDAIRES CONTENANT DE L'ACETAL

(30) Priorität: 26.07.2002 DE 10234016
(43) Veröffentlichungstag der Anmeldung: 27.04.2005
(73) Patentinhaber: BASF SE, 67056 Ludwigshafen (DE)
(72) Erfinder: WARTINI, Alexander, 69120 Heidelberg (DE); SIRCH, Tilman, 67105 Schifferstadt (DE); MELDER, Johann-Peter, 67459 Böhl-Iggelheim (DE); DERNBACH, Matthias, 69221 Dossenheim (DE)
(86) Internationale Anmeldenummer: PCT/EP2003/007870
(87) Internationale Veröffentlichungsnummer: WO 2004/013074

(56) Entgegenhaltungen:
- EP-A- 1 178 030
- GB-A- 1 291 335
- US-A- 6 096 905

## Beschreibung

Die vorliegende Erfindung betrifft ein Verfahren zur Ausbeuteerhöhung bei der Herstellung von durch Kondensation von Formaldehyd mit einem höheren Aldehyd erhaltenen mehrwertigen Alkoholen durch Zersetzung von bei der Herstellung gebildeten Acetalen in einer durch Aufarbeitung erhaltenen Hochsiederfraktion mit einem Wassergehalt von 20 bis 90 Gew.-% durch Säurebehandlung.

Mehrwertige Alkohole werden in großem Maßstab durch Kondensation von Formaldehyd mit höheren, CH-aciden Aldehyden oder mit Wasser und Acrolein bzw. 2-Alkylacroleinen erhalten. Dabei unterscheidet man bei dieser Reaktion zwischen zwei prinzipiellen Durchführungsvarianten.

Zum einen ist dies das sogenannte Cannizarro-Verfahren, das wiederum unterteilt wird in das anorganische und das organische Cannizzaro-Verfahren. Bei der anorganischen Variante setzt man einen Überschuß an Formaldehyd mit dem entsprechenden Alkanal in Gegenwart von stöchiometrischen Mengen einer anorganischen Base wie NaOH oder Ca(OH)₂ um. Das in der ersten Stufe gebildete Methylolalkanal reagiert in der zweiten Stufe mit dem überschüssigen Formaldehyd in einer Disproportionierungsreaktion zum mehrwertigen Alkohol und dem Formiat der entsprechenden Base, also etwa zu Natrium- oder Kalziumformiat. Der Anfall dieser Salze stellte einen Nachteil dar, da sie schwierig vom Reaktionsprodukt abzutrennen sind, und außerdem ein Äquivalent Formaldehyd verloren geht.

Bei dem organischen Cannizzaro-Verfahren wird anstelle einer anorganischen Base ein tertiäres Alkylamin eingesetzt. Es fällt als unerwünschtes Nebenprodukt Trialkylammoniumformiat an. Somit geht auch hier ein Äquivalent des Formaldehyds verloren.

Die Nachteile des Cannizzaro-Verfahrens werden bei dem sogenannten Hydrierverfahren vermieden. Dabei bringt man Formaldehyd mit dem entsprechenden Aldehyd in Gegenwart von katalytischen Mengen eines Amins zur Reaktion. Damit wird erreicht, daß die Reaktion auf der Stufe des alkylolierten Aldehyds anhält. Nach Abtrennung des Formaldehyds wird das Reaktionsgemisch, das neben dem erwähnten alkylolierten Aldehyd noch geringe Mengen des entsprechenden mehrwertigen Alkohols und von Acetalen der gebildeten Alkohole enthält, einer Hydrierung unterworfen, bei der der gewünschte mehrwertige Alkohol erhalten wird.

Ein besonders effektives Verfahren zur Herstellung von durch Kondensation von Aldehyden mit Formaldehyd erhältlichen Alkoholen wird dabei in der WO 98/28253 beschrieben. Hohe Ausbeuten, verbunden mit den Anfallen geringer Mengen an Koppelprodukten, werden mit diesem Verfahren ermöglicht. Es wird dabei so verfahren, daß der höhere Aldehyd mit der 2- bis 8-fachen Menge Formaldehyd in Gegenwart eines tertiären Amins umgesetzt wird, und man das so erhaltene Reaktionsgemisch in zwei Lösungen auftrennt, wobei eine Lösung das erwähnte vollständig methylolierte Alkanal und die andere Lösung nicht umgesetztes Ausgangsprodukt aufweist. Diese letzte Lösung wird in die Reaktion zurückgeführt. Die Auftrennung erfolgt durch Destillation oder einfaches Abtrennen der wässrigen von der organischen Phase. Die das Produkt enthaltende Lösung wird einer katalytischen und/oder thermischen Behandlung unterworfen, um nicht-vollständig alkylolierte Alkanale in die gewünschten vollständig methylolierten Verbindungen zu überführen. Hierbei entstandenes Nebenprodukt wird durch Destillation abgetrennt, und der so erhaltene Sumpf wird der katalytischen Hydrierung, die zu den mehrwertigen Alkoholen führt, unterworfen.

Beispiele für wichtige, mit den beschriebenen Verfahren hergestellte Alkohole sind Neopentylglycol, Pentaerythrit, Trimethylolethan, Trimethylolbutan und insbesondere Trimethylolpropan (TMP).

Sowohl nach dem Cannizzaro- als auch nach dem Hydrierverfahren dargestellte Alkohole müssen destillativ von Komponenten befreit werden, die leichter flüchtig sind (sogenannte Leichtsieder) bzw. schwerer flüchtig sind als dieser (sogenannte Hochsieder) als auch von im Bereich des Alkohols siedenden Komponenten (sogenannte Mittelsieder). Leichtsieder sind dabei insbesondere Wasser, Methanol und bei Verwendung eines Amins als Katalysator das freie Amin.

Bei den Hochsiedern und Mittelsiedern handelt es sich oft um Verbindungen, die Derivate des hergestellten mehrwertigen Alkohols sind und aus diesem durch Reaktion mit beispielsweise Formaldehyd, Methanol oder auch einem im Verlauf des Verfahrens anfallenden Aldehyd oder Alkohol entstanden sind.

Für die Anwendung des mehrwertigen Alkohols ist insbesondere ein niedriger Gehalt des Alkohols an formaldehydhaltigen Acetalen von Bedeutung.

Unter formaldehydhaltigen Acetalen werden dabei alle Verbindungen verstanden, die sich von Formaldehyd ableiten und das Strukturelement

-O-CH₂-O- (I)

aufweisen und auch als Formale bezeichnet werden können.

Bei der Herstellung mehrwertiger Alkohole treten formaldehydhaltige Acetale der allgemeinen Formeln (IIa) oder (IIb) 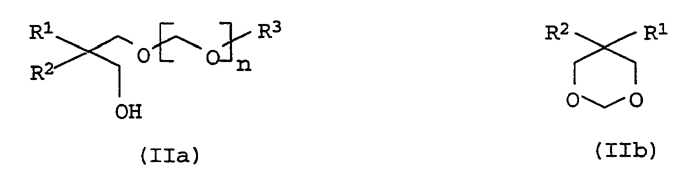 auf, in denen
- R¹, R²: unabhängig voneinander Wasserstoff, C₁- bis C₁₀-Alkyl, C₁-bis C₁₀-Hydroxyalkyl, Carboxyl oder C₁- bis C₄-Alkoxycarbonyl, bevorzugt C₁- bis C₁₀-Alkyl und C₁- bis C₁₀-Hydroxyalkly,
- R³: Wasserstoff, C₁- bis C₁₀-Alkyl, bevorzugt C₁- bis C₈-, besonders bevorzugt C₁- bis C₅-Alkyl, oder C₁- bis C₁₀-Hy droxyalkyl, bevorzugt C₁- bis C₈-, besonders bevorzugt C₁- bis C₅-Alkyl, und
- n: eine ganze Zahl von 1 bis 4, bevorzugt von 1 bis 3 und besonders bevorzugt 1 bis 2,
bedeuten und die Alkylreste jeweils verzweigt oder unverzweigt sein können.

Beispiele für R¹ und R² sind Wasserstoff, Methyl, Ethyl, iso-Propyl, n-Propyl, n-Butyl, iso-Butyl, sek-Butyl, tert-Butyl, n-Pentyl, n-Hexyl, n-Heptyl, n-Octyl, n-Decyl, Hydroxymethyl, Carboxyl, Methoxycarbonyl, Ethoxycarbonyl oder n-Butoxycarbonyl, bevorzugt Wasserstoff, Hydroxymethyl, Methyl und Ethyl, besonders bevorzugt Hydroxymethyl, Methyl und Ethyl.

Beispiele für R³ sind Wasserstoff, Methyl, Ethyl, n-Propyl, n-Butyl, 2-Methylpropyl, 2-Methylbutyl, 2-Ethyl-3-hydroxypropyl, 2-Methyl-3-hydroxypropyl, 2,2-Bis(hydroxymethyl)butyl, 2,2-Bis(hydroxymethyl)propyl, 2,2-Dimethyl-3-hydroxypropyl, 3-Hydroxypropyl, 3-Hydroxy-2-(hydroxymethyl)propyl oder 3-Hydroxy-2,2-bis(hydroxymethyl)propyl.

Typische formaldehydhaltige Acetale sind beispielsweise für den Fall der Synthese des dreiwertigen Alkohols Trimethylolpropan (TMP) aus Formaldehyd und n-Butyraldehyd in Gegenwart katalytischer Mengen an Trialkylamin die nachfolgend genannten TMP-Formaldehyd-Methanol-Acetale (IIIa) und (IIIb), welche im Rohprodukt des Hydrierverfahren zu 0,05 bis 10 Gew.-% enthalten sein können, 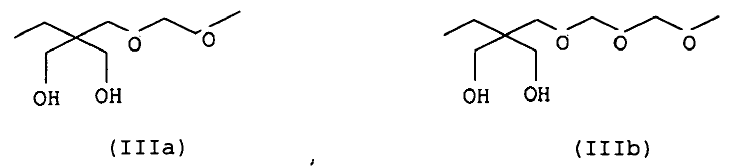 aber auch das lineare bis-TMP-Formal [C₂H₅C(CH₂OH)₂CH₂O]₂CH₂ (IV) und das cyclisehe TMP-Formal

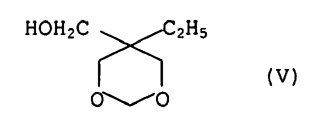

Es ist offensichtlich, dass die Bildung dieser Einheiten des mehrwertigen Alkohols, insbesondere TMP-Einheiten, enthaltenden Acetale unerwünscht ist, da sie die Ausbeute an gewünschtem Produkt deutlich senken und zudem die Anwendungseigenschaften des Produktalkohols negativ beeinflussen. Um diese Nachteile zu vermeiden, ist es wünschenswert, die formaldehydhaltigen Acetale zu spalten und die TMP-Einheiten zurückzugewinnen. Dabei werden in der Literatur verschiedene Verfahren offenbart, um dieses zu erreichen.

EP-A 1 178 030 offenbart die Säurebehandlung der bei der Herstellung von Dimethylolpropan gewonnenen Hochsiederfraktion in Gegenwart von Wasser und deren Rückführung in die Hydrierstufe des Hydrierverfahrens.

In US 6 096 905 ist ein Verfahren offenbart, bei dem eine durch das Cannizarro-Verfahren erhaltene, das lineare bis-TMP-Formal oder das lineare bis-Trimethylolethanformal enthaltende Zusammensetzung mit einem stark sauren Katalysator bei 30 bis 300°C 1/2 bis 8 Stunden lang behandelt wird. Die behandelte Zusammensetzung soll nicht mehr als 15 Gew.-% Wasser enthalten. Der Zusatz eines mit Wasser ein A-zeotrop bildenden Kohlenwasserstoffs wird empfohlen, um den Wassergehalt niedrig zu halten.

Aus DD-A 287 251 ist die Rückgewinnung von Trimethylolpropan aus schwerer als Trimethylolpropan flüchtigen Nebenprodukten durch Säurespaltung bekannt. Die beschriebene Herstellung des Trimethylolpropans erfolgt nach dem Cannizarro-Verfahren. Für die Säu respaltung wird daher ein Maximalgehalt an Alkali bzw. Erdalkaliverbindungen von 0,05 kg/kg angegeben. Wie schon in US 6 096 905 wird auch in der DD-A 287 251 die Wasserkonzentration bei der Säurespaltung der hochsiedenden Nebenprodukte als kritische Größe für den Umsatz angesehen. In der DD 287 251 wird ein möglichst geringer Wassergehalt, maximal jedoch 0,05 kg/kg, empfohlen.

Nachteilig an den aus dem Stand der Technik bekannten Verfahren ist es, dass das stark saure Medium zu Nebenreaktionen führen kann, welche Eigenschaften des gewünschten mehrwertigen Alkohols, wie die Farbzahl nachteilig beeinflussen können.

Die der vorliegenden Erfindung zugrundeliegende Aufgabe ist daher, die Bereitstellung eines Verfahrens, das es erlaubt, die Ausbeuteverluste bei der Herstellung mehrwertiger Alkohole, insbesondere TMP, bevorzugt nach dem Hydrierverfahren, durch die Bildung hochsiedender TMP-haltiger Nebenprodukte zu verringern. Das Verfahren soll effektiv und nicht aufwendig sein, gleichzeitig jedoch die Ausbeute an mehrwertigem Alkohol so verbessern, dass sich ein Einsatz insbesondere beim Hydrierverfahren lohnt.

Die Aufgabe wird gelöst durch ein Verfahren zur Ausbeuteerhöhung bei der Herstellung von durch Aldolisierung von Formaldehyd mit einem höheren Aldehyd in Gegenwart katalytischer Mengen eines tertiären Amins und Hydrieren der so erhaltenen Mono- oder Polymethylolalkanale, erhaltenen mehrwertigen Alkoholen, das folgende Schritte aufweist:
a) destillative Abtrennung der leichter als der mehrwertige Alkohol siedenden Komponenten vom Rohprodukt der Hydrierung der Mono- oder Polymethylolalkanale,
b) Auftrennung des erhaltenen Sumpfprodukts in einer zweiten Destillationsstufe in eine Hochsiederfraktion und eine die Hauptmenge des mehrwertigen Alkohols enthaltende Fraktion,
c) Säurebehandlung der Hochsiederfraktion, wobei der Wassergehalt der Hochsiederfraktion 20 bis 90 Gew.-%, bezogen auf die Gesamtmenge aus Hochsiederfraktion und Wasser, beträgt,
d) Destillation der die Hauptmenge des mehrwertigen Alkohols enthaltenden Fraktion unter Abtrennen der leichter flüchtigen Verbindungen (Mittelsiederfraktion) und Gewinnen von reinem mehrwertigen Alkohol,
dadurch gekennzeichnet, dass die mit säurebehandelte Hochsiederfraktion in die Hydrierung der Mono- oder Polymethylolalkane zum mehrwertigen Alkohol zurückgeführt wird.

Es wurde überraschenderweise gefunden, dass bei hohen Wassergehalten der die Hochsieder enthaltenden Fraktion eine effektive Zersetzung des jeweiligen hochsiedenden Derivats und somit eine deutliche Ausbeuteerhöhung erreicht werden kann. Durch dieses einfache Verfahren ergibt sich eine Ausbeutesteigerung, die bis zu mehreren Prozent betragen kann.

Nach dem erfindungsgemäßen Verfahren kann die Synthese der mehrwertigen Alkohole sowohl nach dem Cannizarro als auch nach dem Hydrierverfahren erfolgt sein.

Das durch das Cannizarro-Verfahren erhaltene Synthesegemisch wird üblicherweise aufgearbeitet, in dem zunächst die als Katalysator dienende anorganische oder organische Base wie NaOH, Ca(OH)₂ oder tertiäres Alkylamin neutralisiert und überschüssiger Aldehyd abgetrennt wird. Anschließend wird der mehrwertige Alkohol vom Formiat der anorganischen oder organischen Base und vom Wasser getrennt (Leichtsieder). Das gewonnene den mehrwertigen Alkohol enthaltende Rohprodukt weist neben Verbindungen der als Katalysator verwendeten Base wie zum Beispiel Salze wie Formiate, Nebenprodukte wie Acetale und Ester und andere höher als der mehrwertige Alkohol siedende Verbindungen auf. Diese Nebenprodukte werden üblicherweise durch Destillation vom Hauptprodukt getrennt, wobei eine schwerer als der mehrwertige Alkohol siedende Fraktion (Hochsiederfraktion) und eine leichter flüchtige Fraktion (Mittelsieder) erhalten wird. Aus dieser durch die an sich bekannte Aufarbeitung erhaltenen Hochsiederfraktionen, die schwerer als der mehrwertige Alkohol siedende Verbindungen wie die vorstehend genannten formaldehydhaltigen Acetale der allgemeinen Formeln (IIa) und (IIb) enthält, werden durch das erfindungsgemäße Verfahren schonend und effektiv die gebundenen Einheiten des mehrwertigen Alkohols zurückgewonnen.

Bevorzugt wird das erfindungsgemäße Verfahren jedoch auf aus Synthesegemischen des Hydrierverfahrens durch Aufarbeitung erhaltene Hochsiederfraktionen angewendet. Der mehrwertige Alkohol wird beim Hydrierverfahren durch Aldolisierung von Formaldehyd mit einem höheren Aldehyd in Gegenwart katalytischer Mengen eines tertiären Amins und Hydrieren der so erhaltenen Mono- oder Polymethylolalkanole, vorzugsweise von Dimethylolbutanol zu Trimethylolpropan, hergestellt wie in der Literatur beschrieben.

Beispiele verschiedener Verfahrensvarianten finden sich dabei in den Anmeldungen DE-A-25 07 461, DE-A-27 02 582 und DE-A-28 13 201, die bereits oben zitiert wurden. Das erfindungsgemäße Verfahren eignet sich besonders zur Ausbeuteerhöhung bei Synthesegemischen, die nach dem in der WO 98/28253 beschriebenen Verfahren hergestellt wurden. Eine kurze Beschreibung dieses Verfahrens findet sich weiter oben. Die Aufarbeitung erfolgt dann in üblicher Weise, wie in der Literatur beschrieben, generell durch Abtrennen von Wasser und anschließender Destillation. Die Hochsiederfraktion kann bei der Aufarbeitung vom Produkt und Mittelsiedern abgetrennt werden, beispielsweise durch Destillation. In einer separaten Stufe wird dann mit der Hochsiederfraktion das erfindungsgemäße Verfahren durchgeführt und der durch Zersetzen der Hochsieder erhaltene Produktalkohol abdestilliert.

Das erfindungsgemäße Verfahren zur Ausbeuteerhöhung lässt sich besonders gut durchführen mit einer durch das in DE-A 199 63 435 beschriebene Verfahren erhaltene Hochsiederfraktion.

Bei dem in DE-A 199 63 435 offenbarten Verfahren wird durch Hydrierung von Mono- oder Polymethylolalkanolen erhaltener mehrwertiger Alkohol, insbesondere aus 2,2-Dimethylolbutanol erhaltenes Trimethylolpropan (TMP), destillativ aufgearbeitet, wobei in der ersten Stufe von dem nach der Hydrierung erhaltenen Rohprodukt Wasser und andere Leichtsieder wie Methanol, Trialkylamin, Trialkylammoniumformiat durch Destillation abgetrennt werden.

Von dem in der ersten Stufe als Sumpfprodukt gewonnenen Gemisch, enthaltend den mehrwertigen Alkohol, insbesondere TMP, Hochsieder und einen Teil leichter als der mehrwertige Alkohol siedende Verbindungen wie zum Beispiel TMP-Formiat, Ethylpropandiol, cyclisches TMP-Formal (im folgenden Mittelsieder genannt) werden, die Hauptmenge des mehrwertigen Alkohols, insbesondere TMP, und die Mittelsieder destillativ von den Hochsiedern getrennt. Die Hochsiederfraktion wird sodann im erfindungsgemäßen Verfahren mit Säure behandelt.

Aus der die Hauptmenge des mehrwertigen Alkohols und die Mittelsieder enthaltenden Fraktion wird unter Abtrennen der Mittelsieder der reine mehrwertige Alkohol gewonnen, der fakultativ einer weiteren Reindestillation zum Gewinnen von mehrwertigen Alkohol mit niedriger Farbzahl unterworfen werden kann.

Aus der Hochsiederfraktion kann nach der erfindungsgemäßen Säurebehandlung der Produktalkohol, bevorzugt durch Destillation, zurückgewonnen werden. In einer besonders bevorzugten Ausführungsform des erfindungsgemäßen Verfahren wird die säurebehandelte Hochsiederfraktionen jedoch direkt ganz oder teilweise in die Hydrierstufe des Hydrierverfahren, d.h. in die Hydrierung der Mono- oder Polymethyl-olalkanale zum mehrwertigen Alkohol, insbesondere des Dimethylolbutanals zum TMP, zurückgeführt. Bei teilweiser Rückführung der säurebehandelten Hochsiederfraktion werden aus dieser vor Rückführung hochsiedende Nebenprodukte über eine destillative Trenneinrichtung oder einen Phasenscheider abgetrennt. Die abgetrennten Nebenprodukte können beispielsweise verbrannt oder anderweitig entsorgt werden.

Diese Verfahrensweise bietet gegenüber der direkten Abtrennung des Produktalkohols aus der säurebehandelten Hochsiederfraktion den Vorteil, dass die Rückbildung hochsiedender Acetale durch Umacetalisierung durch die Hydrierung entstandener Aldehyde vermieden wird und dadurch Ausbeuteerhöhungen im Bereich von mehreren Prozent möglich werden.

In einer besonderen Ausführungsform kann weiterhin die von der Hauptmenge des mehrwertigen Alkohols abgetrennte Mittelsiederfraktion ganz oder teilweise zurückgeführt und mit der Hochsiederfraktion vermischt der Säurebehandlung unterzogen werden. Diese Vermischung mit der Mittelsiederfraktion führt zu einer weiteren Ausbeutesteigerung durch die Spaltung mittelsiedender Acetale. Es wäre auch möglich, die Mittelsiederfraktion anstelle der Hochsiederfraktion nach dem erfindungsgemäßen Verfahren zu behandeln. Erfindungsgemäß wurde jedoch festgestellt, dass die Behandlung der Hochsiederfraktion für sich oder vermischt mit den Mittelsiedern vorteilhaft ist.

Erfindungsgemäß wurde erkannt, das Wassergehalte der Hochsiederfraktion von 20 bis 90 Gew.-%, bevorzugt 40 bis 80 Gew.-%, besonders bevorzugt 70 bis 75 Gew.-%, eine besonders effektive Rückgewinnung von mehrwertigem Alkohol, insbesondere TMP aus der Hochsiederfraktion ermöglicht. Die Einstellung der erfindungsgemäßen Wassergehalte erfolgt durch Wasserzugabe.

Die Menge an Säure, die nach der vorliegenden Erfindung zur Zersetzung der Hochsieder dem Gemisch zugegeben wird, beträgt erfindungsgemäß 0,1 bis 20 Gew.-%, bezogen auf die Gesamtmenge aus Hochsiederfraktion und Wasser, oder das Gemisch aus Hochsiederfraktion und Mittelsiederfraktion und Wasser, bevorzugt 0,1 bis 10 Gew.-%, besonders bevorzugt 0,5 bis 2,5 Gew.-%.

Als Säuren können erfindungsgemäß C₁- bis C₁₂-Carbonsäuren wie Ameisensäure, Essigsäure, Propionsäure, Buttersäure, 2-Ethylhexansäure und Milchsäure, C₂- bis C₁₂-Dicarbonsäuren wie Oxalsäure, Malonsäure, Maleinsäure, Bernsteinsäure und Weinsäure, Sulfonsäuren, Mineralsäuren wie Schwefelsäure, Phosphorsäure und schwefelige Säure, saure Gase in gasförmiger oder wässriger Form wie Kohlendioxid oder Schwefeldioxid oder saure Ionentauscher. Bevorzugt werden Ameisensäure und Phosphorsäure verwendet. Es ist besonders bevorzugt Ameisensäure zu verwenden.

Es wurde erfindungsgemäß erkannt, dass Ameisensäure besonders geeignet ist. Dies ist überraschend, da Ameisensäure im Gegensatz zu den Mineralsäure TMP-Ester bildet und diese TMP-Formiate nur schwer von mehrwertigen Alkohol zu trennen sind.

Insbesondere in der weiter oben beschrieben besonderen Ausführungsform des erfindungsgemäßen Verfahren, in der die säurebehandelte Hochsiederfraktion ganz oder teilweise, vorzugsweise ganz, in die Hydrierung rückgeführt wird, können die Vorteile der Ameisensäure ausgeschöpft werden, denn die im Hydrierverfahren zur Herstellung mehrwertiger Alkohole bevorzugt verwendeten Hydrierkatalysatoren sind in der Lage Formiate zu spalten. Derartige Hydrierkatalysatoren sind beispielsweise in der DE 101 52 527.7 "Verfahren zur Zersetzung von Ammoniumformiaten in polyolhaltigen Reaktionsgemischen" offenbart, auf deren Offenbarung hier ausdrücklich Bezug genommen wird.

Gemäß einer besonders bevorzugten Ausführungsform wird die Hydrierung in Gegenwart des aus der DE-A 198 09 418, auf die hier ausdrücklich Bezug genommen wird, bekannten Katalysators, der einen anorganischen Träger, der TiO₂ enthält, und als Aktivkomponente Kupfer oder ein Gemisch aus Kupfer mit mindestens einem der Metalle, ausgewählt aus der Gruppe Zink, Aluminium, Cer, einem Edelmetall und einem Metall der VIII. Nebengruppe, umfasst und dessen spezifische Kupferoberfläche maximal 10 m²/g beträgt, durchgeführt. Diese Katalysatoren weisen bevorzugt als Träger TiO₂ oder eine Mischung aus TiO₂ und Al₂O₃ oder eine Mischung aus TiO₂ und ZrO₂ oder eine Mischung aus TiO₂, Al₂O₃ und ZrO₂ auf, besonders bevorzugt wird TiO₂ verwendet. Bei der Herstellung dieses Katalysators gemäß DE-A 19809418 kann metallisches Cu-Pulver als weiteres Additiv während der Tablettierung zugesetzt werden, dass die Kupferoberfläche maximal 10 m²/g beträgt.

Die erfindungsgemäße Säurebehandlung der Hochsiederfraktion findet statt bei Temperaturen von 30 bis 180°C, bevorzugt 80 bis 120°C. Dabei werden auf die Hochsiederfraktion bezogene Verweilzeiten gewählt, die von 0,5 bis 10 Stunden, vorzugsweise von 1 bis 6 Stunden, reichen.

Das erfindungsgemäße Verfahren ist nicht ausgeprägt druckabhängig. Die Zersetzung kann im Vakuum, unter Normaldruck oder auch unter Anlegen eines äußeren Drucks durchgeführt werden, bevorzugt unter Normaldruck oder unter dem Eigendruck des Systems. Dabei kann ohne Inertgasatmosphäre, oder mit einer solchen, wie beispielsweise einer Argon- oder Stickstoffatmosphäre, gearbeitet werden.

Das Verfahren ist auf alle mehrwertigen Alkohole anwendbar, die durch Kondensation von Formaldehyd mit höheren Aldehyden unter Zugabe katalytischer Mengen Trialkylamin und nachfolgender Hydrierung hergestellt werden können. Geeignete höhere Aldehyde sind praktisch alle Alkanale mit einem aciden Wasserstoffatom in α-Stellung zur Carbonylgruppe. Es können aliphatische Aldehyde mit 2 bis 24 C-Atomen als Ausgangsmaterialien verwendet werden, die geradkettig oder verzweigt sein oder auch alicyclische Gruppen enthalten können. Ebenso sind araliphatische Aldehyde als Ausgangsstoffe geeignet, vorausgesetzt daß sie eine Methylengruppen in α-Stellung zur Carbonylgruppe enthalten. Im allgemeinen werden Aralkylaldehyde mit 8 bis 24 C-Atomen, vorzugsweise mit 8 bis 12 C-Atomen als Ausgangsmaterialien verwendet, beispielsweise Phenylacetaldehyd. Bevorzugt werden aliphatische Aldehyde mit 2 bis 12 C-Atomen, beispielsweise 3-Ethyl-, 3-n-Propyl-, 3-Isopropyl-, 3-n-Butyl-, 3-Isobutyl-, 3-sek.-Butyl-, 3-tert.-Butyl-butanal sowie entsprechende n-pentanale, -n-hexanale,-n-heptanale; 4-Ethyl-, 4-n-Propyl-, 4-Isopropyl-, 4-n-Butyl-, 4-Isobutyl-, 4-sek.-Butyl-, 4-tert.-Butyl-pentanale, -n-hexanale, -n-heptanale; 5-Ethyl-, 5-n-Propyl-, 5-Isopropyl-, 5-n-Butyl-, 5-Isobutyl-, 5-sek.-Butyl-, 5-tert.-Butyl-n-hexanale, -n-heptanale; 3-Methyl-hexanal, 3-Methyl-heptanal; 4-Methyl-pentanal, 4-Methyl-heptanal, 5-Methyl-hexanal, 5-Methylheptanal; 3,3,5-Trimethyl-n-pentyl-, 3,3-Diethyl-pen-tyl-, 4,4-Diethylpentyl-, 3,3-Dimethyl-n-butyl-, 3,3-Dimethyl-n-pentyl-, 5,5-Dimethylheptyl-, 3,3-Dimethylheptyl-, 3,3,4-Trimethylpentyl, 3,4-Dimethylheptyl-, 3,5-Dimethylheptyl-, 4,4-Dimethylheptyl-, 3,3-Diethylhexyl-, 4,4-Dimethylhexyl-, 4,5-Dimethylhexyl-, 3,4-Dimethylhexyl-, 3,5-Dimethylhexyl-, 3,3-Dimethylhexyl-, 3,4-Diethylhexyl-, 3-Methyl-4-ethylpentyl, 3-Methyl-4-ethylhexyl-, 3,3,4-Trimethylpentyl-, 3,4,4-Trimethylpentyl-, 3,3,4-Trimethylhexyl-, 3,4,4-Trimethylhexyl-, 3,3,4,4,-Tetramethylpentylaldehyd; insbesondere C₂ bis C₁₂-n.-Alkanale.

Besonders bevorzugte mehrwertige Alkohole im Rahmen der vorliegenden Erfindung sind Trimethylolethan, Trimethylolpropan, Trimethylolbutan, Neopentylglykol und Pentaerythrit. Der meist bevorzugte Alkohol ist Trimethylolpropan.

Die Erfindung wird jetzt in den nachfolgenden Beispielen erläutert.

### Beispiele

### Beispiel 1: Herstellung von Roh-TMP

Roh-TMP wurde wie folgt dargestellt:

Eine Apparatur bestehend aus zwei beheizbaren, durch Überlaufrohre miteinander verbundenen Rührkesseln mit einem Fassungsvermögen von insgesamt 72 1 wurde mit frischer, wässriger Formaldehydlösung (4300 g/h in Form der 40 5igen wäßrigen Lösung und n-Butyraldehyd (1800 g/h) und mit frischem Trimethylamin als Katalysator (130 g/h) in Form der 45 %igen wäßrigen Lösung kontinuierlich beschickt. Der Reaktoren wurden dabei auf 40°C temperiert.

Der Austrag wurde direkt in den oberen Teil eines Fallfilmverdampfers mit aufgesetzter Kolonne geleitet und dort bei normalem Druck destillativ in ein leichtsiedendes Kopfprodukt, im wesentlichen enthaltend n-Butyraldehyd, Ethylacrolein, Formaldehyd, Wasser und Trimethylamin, und ein hochsiedendes Sumpfprodukt aufgetrennt.

Das Kopfprodukt wurde kontinuierlich kondensiert und in die oben beschriebenen Reaktoren zurückgeführt.

Das hochsiedende Sumpfprodukt aus dem Verdampfer (ca. 33,5 kg/h) wurde kontinuierlich mit frischem Trimethylamin-Katalysator (50 g/h, in Form der 45 %igen wäßrigen Lösung) versetzt und in einen beheizbaren, mit Füllkörpern versehenen Rohrreaktor mit einem Leervolumen von 12 1 geführt. Der Reaktor war dabei auf 40°C temperiert.

Der Austrag des Nachreaktors wurde kontinuierlich in den oberen Teil einer weiteren Destillationseinrichtung, der Formaldehydabtrennung, gegeben und dort destillativ in ein leichtsiedendes Kopfprodukt, im wesentlichen enthaltend Ethylacrolein, Formaldehyd, Wasser und Trimethylamin, und ein hochsiedendes Sumpfprodukt aufgetrennt. Das leichtsiedende Kopfprodukt (27 kg/h) wurde kontinuierlich kondensiert und in den ersten Rührkessel zurückgeleitet, wohingegen das hochsiedende Sumpfprodukt gesammelt wurde.

Das so erhaltene Sumpfprodukt enthielt neben Wasser im wesentlichen Dimethylolbutyraldehyd, Formaldehyd und Spuren Monomethylolbutyraldehyd. Es wurde dann einer kontinuierlichen Hydrierung unterworfen. Dazu wurde die Reaktionslösung bei 90 bar und 115°C in einem Hauptreaktor in Kreislauf-/Rieselfahrweise und einem nachgeschalteten Nachreaktor in Kreislauffahrweise hydriert. Der Katalysator wurde analog der DE 198 09 418 hergestellt. Er enthielt 24% CuO, 20% Cu und 46% TiO₂. Die verwendete Apparatur bestand aus einem 10 m langen beheizten Nachreaktor (Innendurchmesser: 25 mm). Der Kreislaufdurchsatz betrug 25 1/h Flüssigkeit, der Reaktorzulauf wurde auf 4 kg/h eingestellt. Dementsprechend wurden 4 kg/h Hydrieraustrag erhalten.

Das nach der Hydrierung erhaltene Gemisch wurde entsprechend den Beispielen 2 und 3 der DE 199 63 435 beschriebenen Methode destillativ aufgearbeitet.

Hierbei wird das nach einer Wasserabtrennung erhaltene Gemisch in eine höher als TMP siedende Fraktion, hier Hochsiederfraktion genannt, und eine leichter als TMP siedende Fraktion aufgetrennt, hier als Mittelsieder bezeichnet.

Die so gewonnene Hochsiederfraktion setzt sich im wesentlichen aus folgenden Verbindungen zusammen: 45% TMP-DMB-Acetal, 10% lineares bis-TMP-Formal (IV), 10-25% TMP und 20-35% unbekannte Hochsieder auf.

Die erhaltene Mittelsiederfraktion setzt sich im wesentlichen aus folgenden Verbindungen zusammen: 50% bestehen aus TMP und TMP-Formiat, 10% des cyklischen TMP-Formals (V), 5-10% TMP-Formaldehydacetale (IIa), 5% 2-Ethyl-Propandiol und ca. 20% sind unbekannte Mittelsieder.

### Beispiele 2 bis 11

Alle Versuche bis 100°C wurden in einer Rührapparatur bei Normaldruck unter Stickstoff durchgeführt. Versuche oberhalb von 100°C wurden in einem Autoclaven unter Stickstoffdruck (50 bar) durchgeführt.

Die Analytik erfolgte mit Hilfe von Gaschromatographie (GC) an einer DB5-Säule von J&W Scientific (30 m, 0,32 mm, 1µm), Injektor: 300°C, 90°C bei 15 K pro Minute. Die Detektion erfolgte mit FID.

### Beispiele 2 bis 9

100 g Hochsiederfraktion, wie in Beispiel 1 beschrieben hergestellt, wurden mit der in der Tabelle 1 beschriebenen Menge an Wasser und Ameisensäure versetzt und auf die angegebene Temperatur unter Rühren und Schutzgas erhitzt. Bei Temperaturen oberhalb 100°C wurde die Reaktion im Autoclaven durchgeführt. Die pH-Werte lagen bei allen Versuchen zwischen 2 und 3. Die Zunahmen an Dimethylolbutanol (DMB) beziehungsweise Trimethylolpropan (TMP) wurden gaschromatographisch bestimmt und sind, bezogen auf einen Vergleich mit gleicher Wassermenge ohne Säure bei sonst gleichen Versuchsbedingungen, als Ausbeutezunahme in GC-Flächenprozent (GC-F1.-%) angegeben. Die Ergebnisse sind in Tabelle 1 zusammengefasst.

**Tabelle 1**

| Nr. | Temp. [°C] | Vers.Dauer [h] | Wassermenge | | HCOOH-Menge [Gew.-%]¹ | Zunahme an DMB [GC FI.-%] | Zunahme an TMP [GC FI.-%] |
|---|---|---|---|---|---|---|---|
| | | | [g] | [Gew.-%]¹ | | | |
| 2 | 80 | 6 | 0 | 0 | 5,0 | 0 | -3,3 |
| 3 | 120 | 4 | 0 | 0 | 5,0 | 0 | -6,4 |
| 4 | 100 | 6 | 100 | 50 | 10,0 | 3,4 | 7,3 |
| 5 | 100 | 6 | 100 | 50 | 2,5 | 4,1 | 18,9 |
| 6 | 100 | 6 | 30 | 23 | 2,5 | 2,6 | 5,5 |
| 7 | 100 | 6 | 70 | 41 | 2,5 | 3,6 | 15,8 |
| 8 | 100 | 6 | 300 | 75 | 1,0 | 9,4 | 31,8 |
| 9 | 100 | 6 | 500 | 83 | 1,0 | 5,3 | 36,3 |

| | | | | | | | |
|---|---|---|---|---|---|---|---|
| ¹ Gew.-% beziehen sich auf die Gesamtmenge aus Hochsiederfraktion und Wasser | | | | | | | |

### Beispiel 10

100 g Mittelsiederfraktion, wie in Beispiel 1 erhalten, wurden mit der in der Tabelle beschriebenen Menge an Wasser und Ameisensäure versetzt und auf die angegebene Temperatur unter Rühren und Schutzgas erhitzt. Die Auswertung mit GC-Analytik zeigt den Anstieg an Trimethylolpropan (TMP) und 2,2'-Dimethylolbutanal (DMB). Der pH-Wert lag bei 2,4. Das Ergebnis ist in Tabelle 2 zusammengefasst.

**Tabelle 2**

| Nr. | Temp. [°C] | Vers.Dauer [h] | Wassermenge | | HCOOH-Menge [Gew.-%]¹ | Zunahme an DMB [GC FI.-%] | Zunahme an TMP [GC FI.-%] |
|---|---|---|---|---|---|---|---|
| | | | [g] | [Gew.-%]¹ | | | |
| 10 | 100 | 6 | 100 | 50 | 1,0 | 0 | 25,9 |

| | | | | | | | |
|---|---|---|---|---|---|---|---|
| ¹ Gew.-% beziehen sich auf die Gesamtmenge aus Hochsiederfraktion und Wasser | | | | | | | |

### Beispiel 11

50 g Hochsiederfraktion wurden mit 50 g Mittelsiederfraktion, jeweils erhalten wie in Beispiel 1 beschrieben, gemischt. Das Gemisch wurde mit der in der Tabelle 3 beschriebenen Menge an Wasser und Ameisensäure versetzt und auf die angegebene Temperatur unter Rühren und Schutzgas erhitzt. Die Auswertung mit GC-Analytik zeigt den Anstieg an Trimethylolpropan (TMP) und 2,2'-Dimethylolbutanal (DMB). Der pH-Wert liegt bei 2,1.

**Tabelle 3**

| Nr. | Temp. [°C] | Vers.Dauer [h] | Wassermenge | | HCOOH-Menge [Gew.-%]¹ | Zunahme an DMB [GC FI.-%] | Zunahme an TMP [GC FI.-%] |
|---|---|---|---|---|---|---|---|
| | | | [g] | [Gew.-%]¹ | | | |
| 11 | 100 | 6 | 300 | 75 | 1,0 | 3,9 | 15,1 |

| | | | | | | | |
|---|---|---|---|---|---|---|---|
| ¹ Gew.-% beziehen sich auf die Gesamtmenge aus Hochsiederfraktion und Wasser | | | | | | | |

## Patentansprüche

1. Verfahren zur Ausbeuteerhöhung bei der Herstellung von durch Aldolisierung von Formaldehyd mit einem höheren Aldehyd in Gegenwart katalytischer Mengen eines tertiären Amins und Hydrieren der so erhaltenen Mono- oder Polymethylolalkanale, erhaltenen mehrwertigen Alkoholen, das folgende Schritte aufweist:
a) destillative Abtrennung der leichter als der mehrwertige Alkohol siedenden Komponenten vom Rohprodukt der Hydrierung der Mono- oder Polymethylolalkanale,
b) Auftrennung des erhaltenen Sumpfprodukts in einer zweiten Destillationsstufe in eine Hochsiederfraktion und eine die Hauptmenge des mehrwertigen Alkohols enthaltende Fraktion,
c) Säurebehandlung der Hochsiederfraktion, wobei der Wassergehalt der Hochsiederfraktion 20 bis 90 Gew.-%, bezogen auf die Gesamtmenge aus Hochsiederfraktion und Wasser, beträgt,
d) Destillation der die Hauptmenge des mehrwertigen Alkohols enthaltenden Fraktion unter Abtrennen der leichter flüchtigen Verbindungen (Mittelsiederfraktion) und Gewinnen von reinem mehrwertigen Alkohol,
**dadurch gekennzeichnet, dass** die mit säurebehandelte Hochsiederfraktion in die Hydrierung der Mono- oder Polymethylolalkane zum mehrwertigen Alkohol zurückgeführt wird.

2. Verfahren nach Anspruch 1, **dadurch gekennzeichnet, dass** die durch Destillation aus der die Hauptmenge der mehrwertigen Alkohols enthaltenden Fraktion abgetrennte Mittelsiederfraktion vor der Säurebehandlung ganz oder teilweise mit der Hochsiederfraktion vermischt wird.

3. Verfahren nach einem der Ansprüche 1 oder 2, **dadurch gekennzeichnet, dass** die Säurekonzentration von 0,1 Gew.-% bis 20 Gew.-%, bezogen auf die Gesamtmenge aus Hochsiederfraktion oder dem Gemisch aus Hochsiederfraktion und Mittelsiederfraktion und Wasser, beträgt.

4. Verfahren nach einem der Ansprüche 1 bis 3, **dadurch gekennzeichnet, dass** die Säure ausgewählt ist aus C₁- bis C₁₂-Carbonsäuren, C₂- bis C₁₂-Dicarbonsäuren, Sulfonsäuren, Mineralsäuren, Kohlendioxid, Schwefeldioxid und sauren Ionentauschern.

5. Verfahren nach einem der Ansprüche 1 bis 4, **dadurch gekennzeichnet, dass** Ameisensäure verwendet wird.

6. Verfahren nach einem der Ansprüche 1 bis 5, **dadurch gekennzeichnet, dass** die mehrwertigen Alkohole ausgewählt sind aus der Gruppe Trimethylolethan, Trimethylolpropan, Trimethylolbutan, Neopentylgylkol und Pentaerythrit.

7. Verfahren nach einem der Ansprüche 1 bis 6, **dadurch gekennzeichnet, dass** der mehrwertige Alkohol Trimethylolpropan ist.

## Claims

1. A process for increasing the yield in preparing polyhydric alcohols obtained by aldolizing formaldehyde with a higher aldehyde in the presence of catalytic amounts of a tertiary amine and hydrogenating the mono- or polymethylolalkanals obtained in this way, which has the following steps:
a) distillative removal of the components having lower boiling points than the polyhydric alcohol from the crude product of the hydrogenation of the mono- or polymethylolalkanals,
b) separation of the resulting bottom product in a second distillation stage into a high boiler fraction and a fraction comprising the majority of the polyhydric alcohol,
c) acid treatment of the high boiler fraction, the water content of the high boiler fraction being from 20 to 90% by weight, based on the total amount of high boiler fraction and water,
d) distillation of the fraction comprising the majority of the polyhydric alcohol to remove the more volatile compounds (medium boiler fraction) and recovery of pure polyhydric alcohol,
wherein the acid-treated high boiler fraction is recycled into the hydrogenation of the mono- or polymethylolalkanals to the polyhydric alcohol.

2. The process according to claim 1, wherein some or all of the medium boiler fraction removed from the fraction comprising the majority of the polyhydric alcohol by distillation is mixed with the high boiler fraction before the acid treatment.

3. The process according to either of claims 1 and 2, wherein the acid concentration is from 0.1% by weight to 20% by weight, based on the total amount of high boiler fraction or the mixture of high boiler fraction and middle boiler fraction and water.

4. The process according to any of claims 1 to 3, wherein the acid is selected from C₁- to C₁₂-carboxylic acids, C₂- to C₁₂-dicarboxylic acids, sulfonic acids, mineral acids, carbon dioxide, sulfur dioxide and acidic ion exchangers.

5. The process according to any of claims 1 to 4, wherein formic acid is used.

6. The process according to any of claims 1 to 5, wherein the polyhydric alcohols are selected from the group of trimethylolethane, trimethylolpropane, trimethylolbutane, neopentyl glycol and pentaerythritol.

7. The process according to any of claims 1 to 6, wherein the polyhydric alcohol is trimethylolpropane.

## Revendications

1. Procédé pour augmenter le rendement lors de la préparation d'alcools polyvalents obtenus par aldolisation de formaldéhyde avec un aldéhyde supérieur en présence de quantités catalytiques d'une amine tertiaire et hydrogénation des mono- ou polyméthylolalcanals ainsi obtenus, comprenant les étapes suivantes :
a) séparation par distillation des composants plus volatils que l'alcool polyvalent à partir du produit brut de l'hydrogénation des mono- ou polyméthylolalcanals,
b) séparation de la fraction de queue obtenue, dans une deuxième étape de distillation, en une fraction à haut point d'ébullition et une fraction contenant la majeure partie de l'alcool polyvalent,
c) traitement par un acide de la fraction à haut point d'ébullition, la teneur en eau de la fraction à haut point d'ébullition étant de 20 à 90 % en poids, par rapport à la quantité totale de la fraction à haut point d'ébullition et de l'eau,
d) distillation de la fraction contenant la majeure partie de l'alcool polyvalent avec séparation des composés volatils à plus bas point d'ébullition (fraction à moyen point d'ébullition) et obtention d'alcool polyvalent pur,
**caractérisé en ce que** la fraction à haut point d'ébullition traitée à l'acide est renvoyée dans l'hydrogénation des mono- ou polyméthylolalcanals en alcool polyvalent.

2. Procédé suivant la revendication 1, **caractérisé en ce que** la fraction à point d'ébullition moyen séparée par distillation de la fraction contenant la majeure partie de l'alcool polyvalent est mélangée en tout ou en partie à la fraction à haut point d'ébullition avant le traitement par l'acide.

3. Procédé suivant la revendication 1 ou 2, **caractérisé en ce que** la concentration d'acide est de 0,1 % en poids à 20 % en poids, par rapport à la quantité totale de la fraction à haut point d'ébullition ou du mélange de fraction à haut point d'ébullition et de fraction à point d'ébullition moyen et d'eau.

4. Procédé suivant l'une quelconque des revendications 1 à 3, **caractérisé en ce que** l'acide est choisi parmi des acides carboxyliques en C₁ à C₁₂, des acides dicarboxyliques en C₂ à C₁₂, des acides sulfoniques, des acides minéraux, du dioxyde de carbone, du dioxyde de soufre et des échangeurs d'ions acides.

5. Procédé suivant l'une quelconque des revendications 1 à 4, **caractérisé en ce que** l'on utilise de l'acide formique.

6. Procédé suivant l'une quelconque des revendications 1 à 5, **caractérisé en ce que** les alcools polyvalents sont choisis dans le groupe formé par le triméthyloléthane, le triméthylolpropane, le triméthylolbutane, le néopentylglycol et le pentaérythritol.

7. Procédé suivant l'une quelconque des revendications 1 à 6, **caractérisé en ce que** l'alcool polyvalent est du triméthylolpropane.
